(19) Europäisches Patentamt
European Patent Office
Office européen des brevets

(11) EP 1 688 453 B1

(12) # EUROPÄISCHE PATENTSCHRIFT

(45) Veröffentlichungstag und Bekanntmachung des Hinweises auf die Patenterteilung:
**23.07.2008 Patentblatt 2008/30**

(21) Anmeldenummer: **06001168.1**

(22) Anmeldetag: **20.01.2006**

(51) Int Cl.:
*C08G 77/388* (2006.01) *C08G 77/26* (2006.01)
*C08G 77/04* (2006.01) *A61K 8/893* (2006.01)
*A61Q 5/12* (2006.01) *A61Q 17/04* (2006.01)
*C11D 1/62* (2006.01) *C11D 3/37* (2006.01)

(54) **UV-Licht absorbierende quaternäre Polysiloxane**

UV light absorbing quaternary polysiloxanes

Polysiloxanes quaternaires absorbant les rayons UV

(84) Benannte Vertragsstaaten:
**DE ES FR GB IT PL**

(30) Priorität: **02.02.2005 DE 102005004706**

(43) Veröffentlichungstag der Anmeldung:
**09.08.2006 Patentblatt 2006/32**

(73) Patentinhaber: **Evonik Goldschmidt GmbH**
**45127 Essen (DE)**

(72) Erfinder:
- **Pascaly, Matthias, Dr.**
**48163 Münster (DE)**
- **Ferenz, Michael, Dr.**
**45147 Essen (DE)**
- **Grüning, Burghard, Dr.**
**45134 Essen (DE)**
- **Leidreiter, Holger, Dr.**
**45529 Hattingen (DE)**
- **Messinger, Erika**
**34454 Arolsen, Schmillinghausen (DE)**
- **Zündorff, Astrid**
**45472 Mülheim an der Ruhr (DE)**

(74) Vertreter: **Lang, Arne**
**Evonik Degussa GmbH**
**DG-IPM-PAT**
**Bau 1042 / PB 15**
**Paul-Baumann-Strasse 1**
**45764 Marl (DE)**

(56) Entgegenhaltungen:
**EP-A- 0 606 634**
**EP-A- 1 285 943**
**US-A- 5 610 257**
**US-A- 5 837 792**
**US-A1- 2003 133 886**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 688 453 B1

## Beschreibung

Die Erfindung betrifft UV-Licht absorbierende quaternäre Polysiloxane und deren Verwendung in Formulierungen zur Textilpflege und Kosmetikzubereitungen.

Es ist bekannt, dass UV-Licht der Wellenlängen von 200 bis 400 nm für das Ausbleichen und die Schädigung von Textilien, synthetischen Fasern und natürlichen Fasern (z.B. Wolle, Baumwolle und Haare) verantwortlich ist.

Aus diesem Grund besteht ein wachsender Bedarf an Verbindungen, die die Textilien, synthetischen Fasern und natürlichen Fasern vor der UV-Strahlung abschirmen oder eine Kontrolle über den Grad der Schädigung erlauben.

Es ist daher wünschenswert, Verbindungen bereitzustellen, die eine Kontrolle über die UV-Strahlung ausüben können, der die Textilien bzw. die synthetischen oder natürlichen Fasern ausgesetzt werden.

Aus der Literatur ist eine große Anzahl von Verbindungen bekannt, die für den UV-Lichtschutz von Fasern, Farbstoffen und Pigmenten eingesetzt werden. Solche Verbindungen werden typischerweise direkt bei der Herstellung der Faser eingesetzt.

Diese Verbindungen zeigen aber keinen pflegenden bzw. weichmachenden Effekt. Zusätzlich fehlt ihnen oft die Haftung auf der Faseroberfläche, so dass schon nach wenigen Waschvorgängen der UV-Schutz auf der Faser verlorengeht.

Es ist daher wünschenswert, Verbindungen bereitzustellen, die einen pflegenden Effekt auf natürliche oder synthetische Fasern ausüben, eine hohe Substantivität auf der Faser zeigen und zusätzlich einen Schutz vor Schädigungen durch mechanische und/oder optische (z.B. UV-Licht) Einflüsse bieten.

Um auch nach mehrmaliger Wäsche einen hinreichenden UV-Schutz bereitstellen zu können, sollten die Verbindungen auch in Weichspülformulierungen einarbeitbar sein und während des Weichspülvorgangs auf die Faser aufziehen.

Quaternäre Polysiloxane sind ebenfalls aus der Literatur bekannt und zum Beispiel in den Patenten EP-A-0 282 720 und DE-A-37 19 086 beschrieben. Solche Verbindungen sind besonders für ihre konditionierenden Eigenschaften in der Haarkosmetik und für ihre weichmachenden und pflegenden Effekte in der Textilbehandlung bekannt. Verbindungen dieser Art werden weiterhin eingesetzt, um die Elastizität bzw. die Reißfestigkeit von Textilien zu erhöhen und deren Falten- bzw. Knitterbildung zu verringern und/oder das spätere Bügeln zu erleichtern ("easy ironing") (WO-A-01/25385, WO-A-01/25382, WO-A-01/25381, WO-A-01/25380, WO-A-99/55953). Verbindungen, wie sie in den Patenten EP-B-0 282 720 und DE-C-37 19 086 beschrieben sind, zeigen jedoch keinen Schutz vor dem schädlichen Einfluss der UV-Strahlung.

In DE-A-101 41 356 bzw. US-B-6 630 132 ist bereits die Kombination von quaternären mit UV-absorbierenden Gruppen, gebunden an ein Silicon Backbone, beschrieben. Nachteilig gemäß diesem Stand der Technik ist es, dass diese Verbindungen pro UV-absorbierender Gruppe ein quaternäres Stickstoff-Atom enthalten. Somit ergibt sich bei hoher UV-Absorption eine hohe kationische Ladungsdichte. Das Verhältnis von Ladungsdichte zu UV-Absorption kann also nicht beliebig variiert werden.

Die komplexe Aufgabe der vorliegenden Erfindung bestand nun darin, Silicon-basierende Verbindungen bereitzustellen, in denen der Gehalt an quaternären und UV-absorbierenden Gruppen unabhängig voneinander beliebig variiert werden kann. Die Substantivität dieser Verbindungen für natürliche oder synthetische Fasern (z.B. Wolle, Baumwolle oder Haare) oder andere Oberflächen (z.B. der Haut) soll nach Wunsch eingestellt werden können, durch die Siliconkette soll ein glättender, pflegender und weichmachender Effekt auf natürliche oder synthetische Fasern erzielt werden und zusätzlich sollen Schädigungen durch mechanische und/oder optische Einflüsse (z.B. UV-Licht) vermindert werden.

Überraschenderweise wurde nun gefunden, dass die vorgenannte Aufgabe gelöst wird durch die unabhängige Kombination quaternärer und UV-absorbierender Gruppen an einem Silicone Backbone.

Ein Gegenstand der Erfindung sind daher UV-Licht absorbierende quaternäre Polysiloxane der allgemeinen Formel (I)

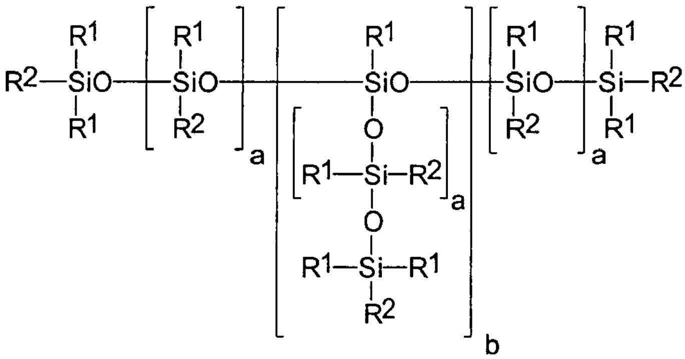

(I)

worin die Reste

$R^1$ gleich oder verschieden sind und jeweils Alkylreste mit 1 bis 22, vorzugsweise 1 bis 4 Kohlenstoffatomen oder Phenylreste bedeuten,

$R^2$ zu einem Teil die Bedeutung der Reste $R^1$ haben können und die übrigen Reste $R^2$ unabhängig voneinander Reste der Formeln (Ia oder Ib)

$$R^2 = -M-S$$

oder

$$R^2 = -M-Z^+ A^-$$

sind, mit der Maßgabe, dass im durchschnittlichen Molekül mindestens zwei Reste $R^2$ jeweils ein Rest der Formel -M-S und $-M-Z^+ A^-$ sind,
worin
S ein einwertiger, für die UV-Absorption verantwortlicher Chromophorrest ist, insbesondere ein Rest der Formel (Ic)

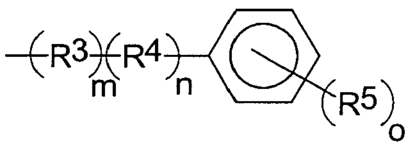

(Ic)

worin
$R^3$

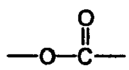

oder

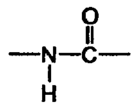

ist,
$R^4$ -CH=CH- ist,
$R^5$ gleich oder verschieden sind und jeweils Wasserstoff, Alkyl-, Haloalkyl-, Halogen-, Phenyl-, Hydroxyl-, Alkoxy-, Amino-, Alkylamino-, Dialkylamino-, Di (hydroxyalkyl) amino- oder Di (poly-alkoxy)aminoreste bedeuten,
m = 0 oder 1,
n = 0 oder 1,
o = 0 bis 5
und
$Z^+$ ein Rest der Formel (Id)

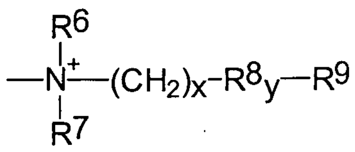

(Id)

ist,
$R^6$, $R^7$ Alkylreste mit 1 bis 22 Kohlenstoffatomen oder Alkenylreste mit 2 bis 22 Kohlenstoffatomen, worin die Alkyl- oder Alkenylreste Hydroxylgruppen aufweisen können,
$R^8$

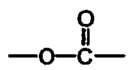

oder

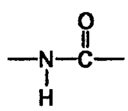

ist,
$R^9$ ein einwertiger Kohlenwasserstoffrest mit 1 bis 22 Kohlenstoffatomen sein kann,
oder
ein Rest nach Formel (Ic) ist,
x = 0 bis 6,
y = 0 oder 1 ist
und
M ein zweiwertiger Kohlenwasserstoffrest mit mindestens 4 Kohlenstoffatomen ist, der eine Hydroxylgruppe aufweist und der durch ein oder mehrere Sauerstoffatome unterbrochen sein kann,

$A^-$ ein anorganisches oder organisches Anion, das von einer üblichen physiologisch verträglichen Säure HA herrührt, ist,

a unabhängig voneinander einen Wert von 1 bis 200 und
b einen Wert von 0 bis 10 hat.

Gegenstand der vorliegenden Erfindung sind UV-Licht absorbierende quaternäre Polysiloxane erhältlich durch Umsetzung von Verbindungen der allgemeinen Formel (II)

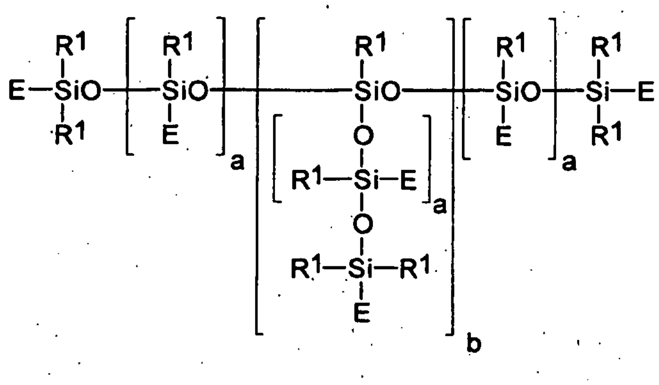

(II)

worin die Reste

$R^1$ gleich oder verschieden sind und jeweils Alkylreste mit 1 bis 22, insbesondere 1 bis 4 Kohlenstoffatomen oder Phenylreste bedeuten,

E zu einem Teil die Bedeutung der Reste $R^1$ haben können und die übrigen Reste E einwertige Reste sind, mit der allgemeinen Formel

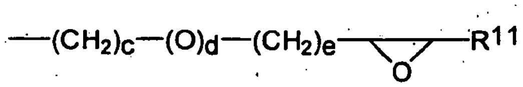

mit
c, e unabhängig voneinander 0 bis 10, insbesondere 1 bis 3, in der Summe ≥ 4,
d 0 oder 1 und
$R^{11}$ H oder ein einwertiger Kohlenwasserstoffrest mit 1 bis 10 Kohlenstoffatomen sein kann,

mit der Maßgabe, dass im durchschnittlichen Molekül mindestens zwei Reste E vorhanden sind,

a unabhängig voneinander einen Wert von 1 bis 200 und
b einen Wert von 0 bis 10 hat,

mit mindestens einer Verbindung der allgemeinen Formel (IIa)

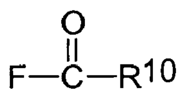

(IIa)

worin

F OH oder $NH_2$ sind,

$R^{10}$ ein einwertiger, für die UV-Absorption verantwortlicher Chromophorrest, der Formel (Ic mit m = 0)

und zusätzlich
mit mindestens einem tertiären Amin der allgemeinen Formel (IIb)

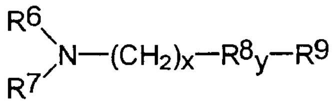

(IIb)

worin

$R^6$, $R^7$ Alkylreste mit 1 bis 22 Kohlenstoffatomen oder Alkenylreste mit 2 bis 22 Kohlenstoffatomen, worin die Alkyl- oder Alkenylreste Hydroxylgruppen aufweisen können,

$R^8$

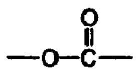

oder

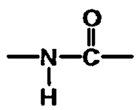

ist,

$R^9$ ein einwertiger Kohlenwasserstoffrest mit 1 bis 22 Kohlenstoffatomen sein kann,

oder
ein Rest nach Formel (Ic) ist,
mit
x = 0 bis 6,
y = 0 oder 1

in an sich bekannter Weise in Gegenwart eines üblichen physiologisch verträglichen organischen oder anorganischen Säureequivalentes HA, bezogen auf tertiäres Amin und bei Temperaturen von 40 bis 120 °C umsetzt, wobei A- ein anorganisches oder organisches Anion, das von einer üblichen physiologisch verträglichen Säure HA herrührt, ist.

Die Mengenverhältnisse der Verbindungen (IIa) und (IIb) sind unabhängig voneinander variierbar von 0,001 : 1 bis 1 : 0,001, insbesondere von 0,1 : 1 bis 1 : 0,1.

Bevorzugte Beispiele für den Rest $R^1$ sind Alkylreste mit 1 bis 22, insbesondere 1 bis 4 Kohlenstoffatomen oder der Phenylrest, wie der Methyl-, Ethyl-, n-Propyl-, iso-Propyl- oder n-Butylrest, iso-Butylrest.

Bevorzugte Beispiele für den Rest M sind:

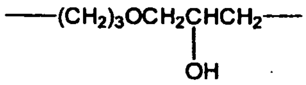 , 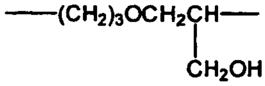 , 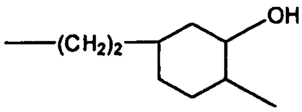 ,

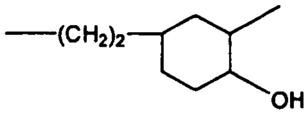 , 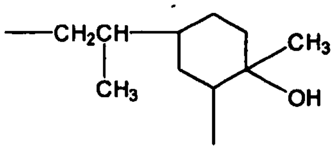 , 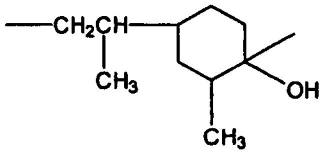 ,

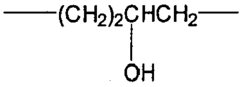 , 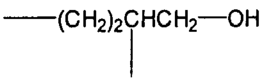 , 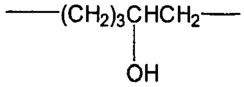 , 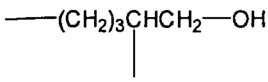 ,

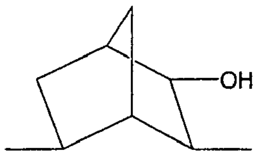 , 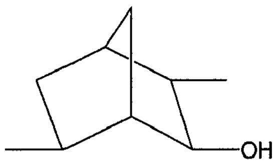 .

Innerhalb der erfindungsgemäßen Verbindungen können die Reste S und $Z^+$ gleiche oder unterschiedliche Bedeutung haben.

Bevorzugte Beispiele für den Rest S sind:

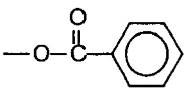 , 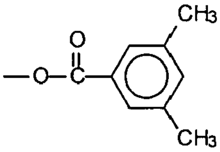 , 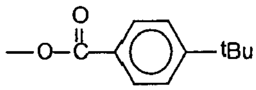 , 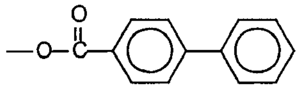 ,

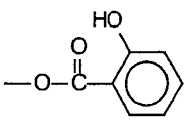 , 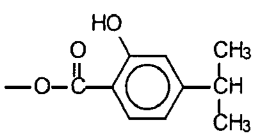 , 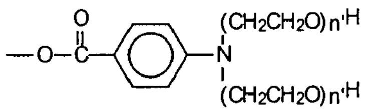 (n'=0-40),

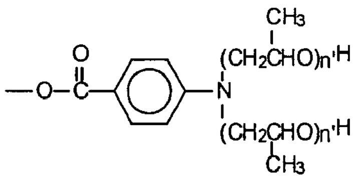

(n‘=1-40),

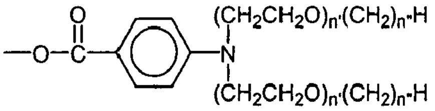

(n′=0-40, n′′=1-22),

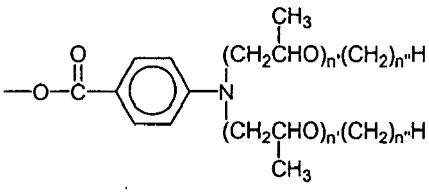

(n′=1-40, n′′=1-22),

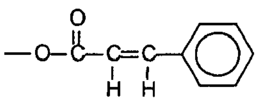

,

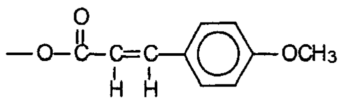

,

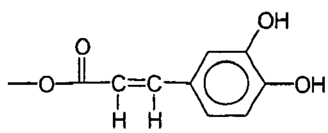

,

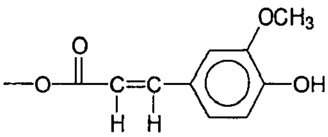

,

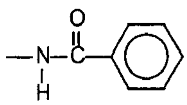

,

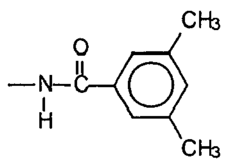

,

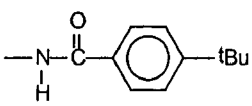

,

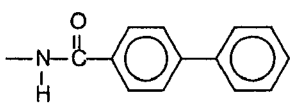

,

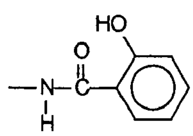

,

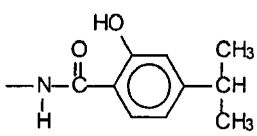

,

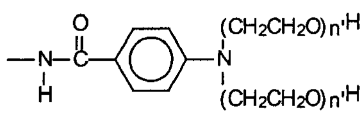

(n‘=0-40),

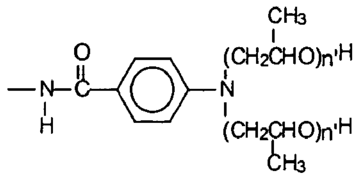

(n'=0-40),

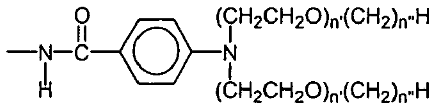

(n'=0-40, n''=1-22),

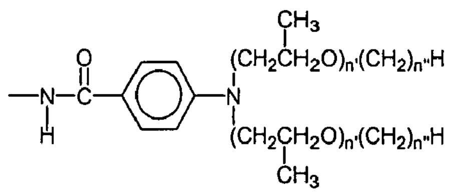

(n'=1-40, n''=1-22),

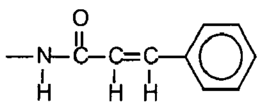

,

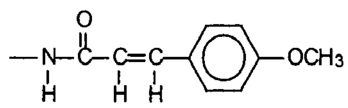

,

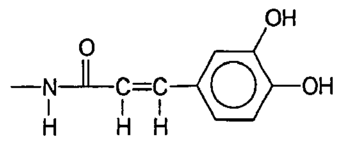

,

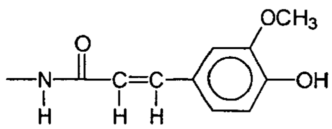

.

Bevorzugte Beispiele für den Rest Z sind Capryldimethylamin, Lauryldimethylamin, Cocodimethylamin, Myristyldimethylamin, Cetyldimethylamin, Stearyldimethylamin, Behenyldimethylamin, Oleyldimethylamin, Capryloylamidopropyldimethylamin, Lauramidopropyldimethylamin, Cocamidopropyldimethylamim, Myristamidopropyldimethylamin, Palmitamidopropyldimethylamin, Stearamidopropyldimethylamin, Behenamidopropyldimethylamin, Oleamidopropyldimethylamin, Undecylenamidopropyldimethylamin, Ricinolamidopropyldimethylamin sowie Guanidinopropyldimethylamin.

Bevorzugte Beispiele für $A^-$, ein anorganisches oder organisches Anion, das von einer üblichen physiologisch verträglichen Säure HA herrührt, sind Acetat-, Chlorid-, Bromid-, Hydrogensulfat-, Sulfat-, Methosulfat-, Ethosulfat-, Citrat-, Tartrat- und Lactationen, sowie Anionen aromatischer Säuren, wie die Anionen der p-Toluolsulfonsäure, Benzoesäure, Salicylsäure, Zimtsäure, 4-Methoxyzimtsäure, 4-Aminobenzoesäure, 4-Bis(hydroxypropyl)-aminobenzoesäure, 4-Bis(polyethoxy)aminobenzoesäure, 4-Dimethylaminobenzoesäure, 3-Imidazol-4-yl-acrylsäure, 2-Phenylbenzimidazol-5-sulfonsäure, 3,3'-(1,4-Phenylendimethin)-bis-(7,7-dimethyl-2-oxo-bicyclo-[2.2.1]heptan-1-methansulfonsäure), 2-Hydroxy-4-methoxybenzophenon-5-sulfonsäure und 3-(4'-Sulfo)-benzyliden-bornan-2-on.

Es ist dem Fachmann geläufig, dass die Verbindungen in Form eines Gemisches mit einer im Wesentlichen durch statistische Gesetze geregelten Verteilung vorliegen. Die Werte für die Indices a und b stellen deshalb Mittelwerte dar.

Bevorzugte Beispiele für UV-Licht absorbierende quaternäre Polysiloxane, sind Verbindungen der Formeln

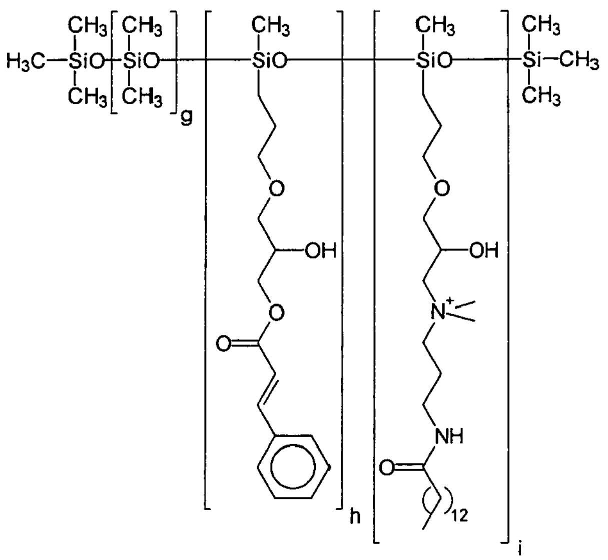

| g | h | i |
|---|---|---|
| 58 | 35 | 5 |
| 73 | 20 | 5 |
| 40 | 8 | 2 |
| 58 | 32 | 8 |
| 90 | 24 | 6 |

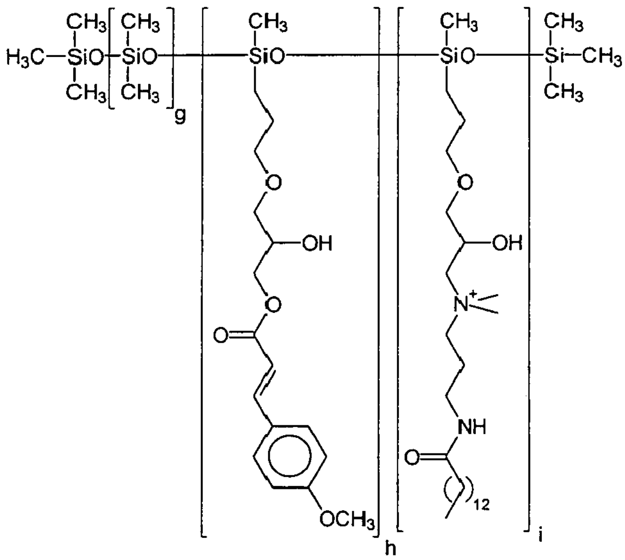

| g | h | i |
|---|---|---|
| 58 | 35 | 5 |
| 73 | 20 | 5 |
| 40 | 8 | 2 |
| 58 | 32 | 8 |
| 90 | 24 | 6 |

Verfahren zur Herstellung der erfindungsgemäßen Verbindungen und die Eigenschaften dieser Verbindungen sind in den folgenden Beispielen näher beschrieben:

<u>Beispiel 1:</u>

Herstellung eines erfindungsgemäßen UV-Licht absorbierenden quaternären Polysiloxans der allgemeinen Formel:

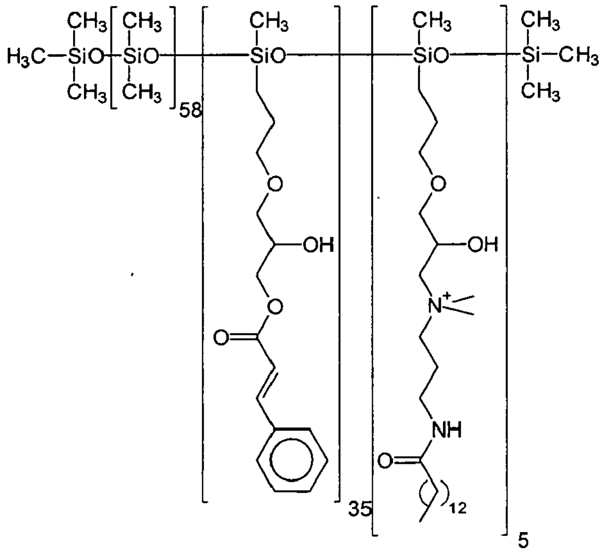

In einem 250 ml-Vierhalskolben, versehen mit Rührer, Tropftrichter, Thermometer und Rückflusskühler, werden 5,5 g (0,0176 Mol) eines tertiären Amins der allgemeinen Formel:

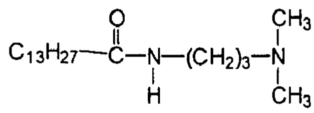

zusammen mit 1,9 g (.0,032 Mol) Essigsäure und 16,5 g Zimtsäure (0,111 mmol) sowie 30 ml Isopropanol vorgelegt. Nach 5 Minuten werden 42 g (0,14 Mol Epoxy) eines Epoxysiloxans der allgemeinen Formel:

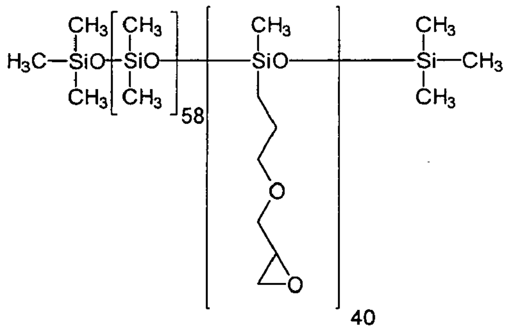

zugetropft, auf Rückflusstemperatur erwärmt und 6 Stunden gerührt. Anschließend wird bei 100 °C im Vakuum destilliert. Es wird ein hochviskoses, gelb-braunes Produkt erhalten.

UV-vis Spektrum Beispiel 1 (0,005 % in Isopropanol):

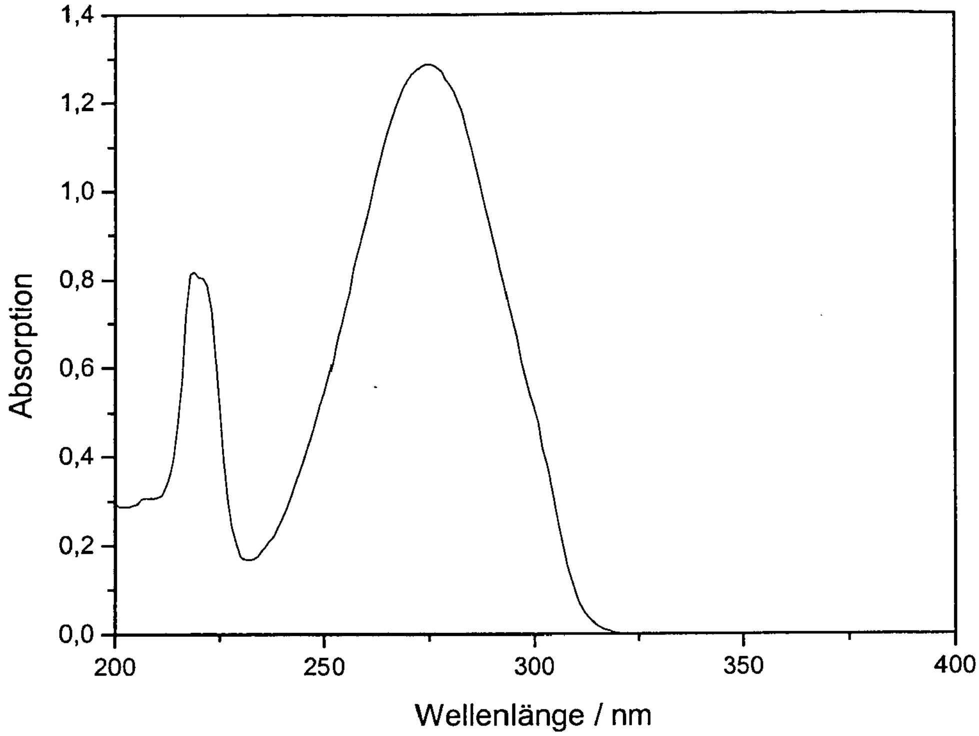


Das Spektrum zeigt die breite charakteristische Absorptionsbande der Zimtsäureeinheiten bei 275 nm. Die Intensität korreliert mit der verwendeten Menge an Zimtsäure.

<u>Beispiel 2:</u>

Herstellung eines erfindungsgemäßen UV-Licht absorbierenden quaternären Polysiloxans der allgemeinen Formel:

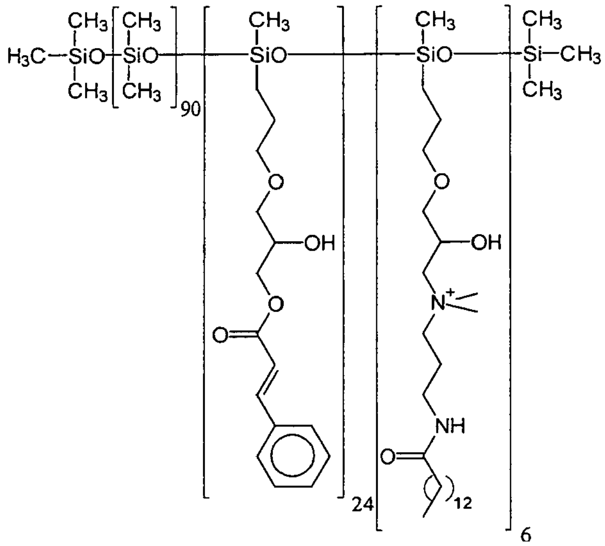

In einem 250 ml-Vierhalskolben, versehen mit Rührer, Tropftrichter, Thermometer und Rückflusskühler, werden 6,3 g (0,02 Mol) eines tertiären Amins der allgemeinen Formel:

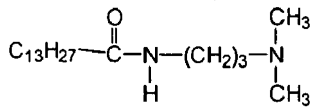

zusammen mit 1,3 g (0,021 Mol) Essigsäure und 11,8 g Zimtsäure (0,08 Mol) sowie 30 ml Isopropanol vorgelegt. Nach 5 Minuten werden 38 g (0,10 Mol Epoxy) eines Epoxysiloxans der allgemeinen Formel:

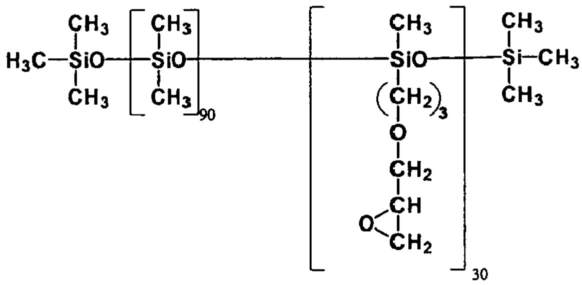

zugetropft, auf Rückflusstemperatur erwärmt und 6 Stunden gerührt. Anschließend wird bei 100 °C im Vakuum destilliert. Es wird ein hochviskoses, gelb-braunes Produkt erhalten (Quarternärer Stickstoff gef.: 1,65 %; theor.: 1,9 %.).

UV-vis Spektrum Beispiel 2 (0,005 % in Isopropanol):

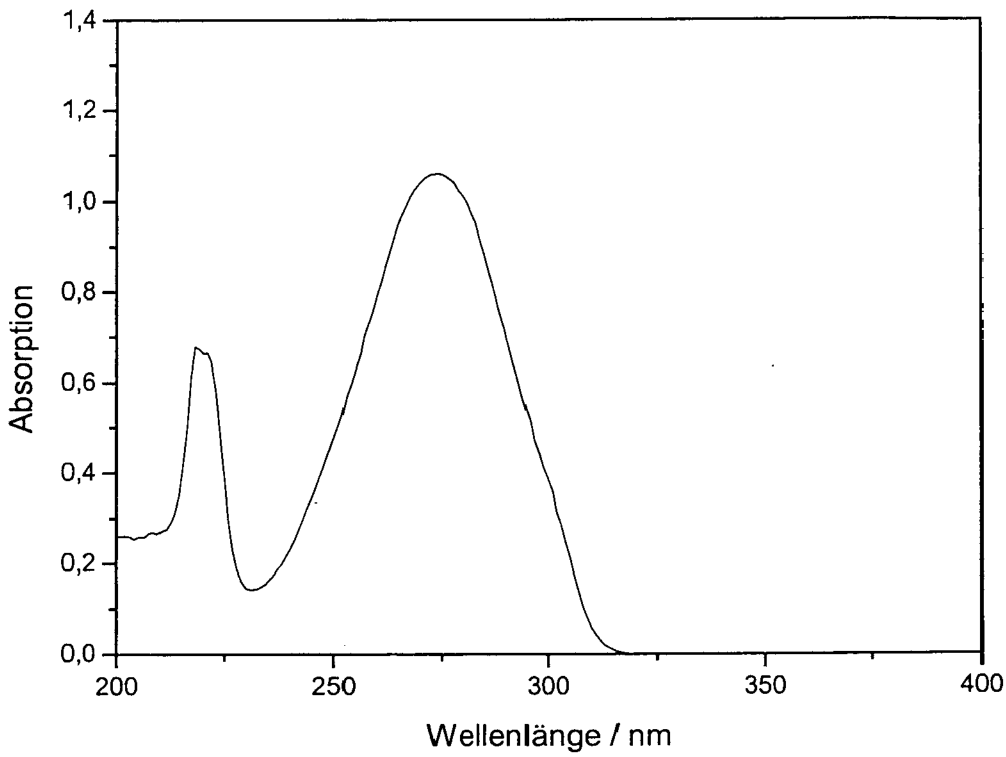


Das Spektrum zeigt die breite charakteristische Absorptionsbande der Zimtsäureeinheiten bei 275 nm. Die

Intensität korreliert mit der verwendeten Menge an Zimtsäure.

Beispiel 3:

Herstellung eines erfindungsgemäßen UV-Licht absorbierenden quaternären Polysiloxans der allgemeinen Formel:

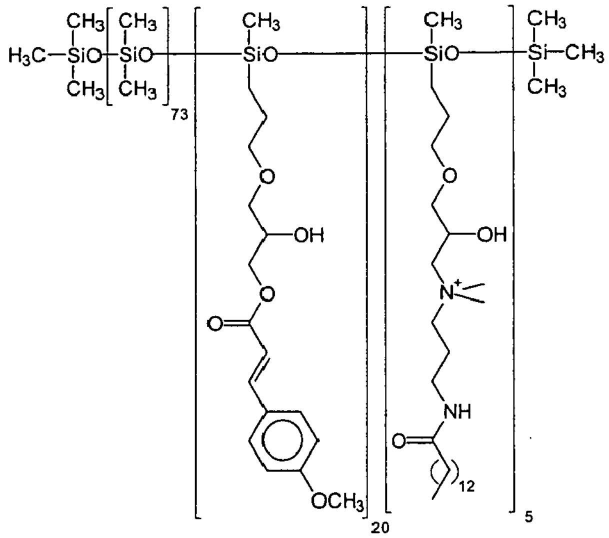

In einem 250 ml-Vierhalskolben, versehen mit Rührer, Tropftrichter, Thermometer und Rückflusskühler, werden 15,8 g (0,05 Mol) eines tertiären Amins der allgemeinen Formel:

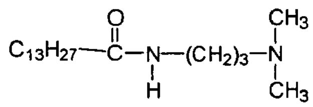

zusammen mit 3,8 g (0,051 Mol) Essigsäure und 35,6 g 4-Methoxyzimtsäure (0,2 Mol) sowie 30 ml Isopropanol vorgelegt. Nach 5 Minuten werden 97,6 g (0,25 Mol Epoxy) eines Epoxysiloxans der allgemeinen Formel:

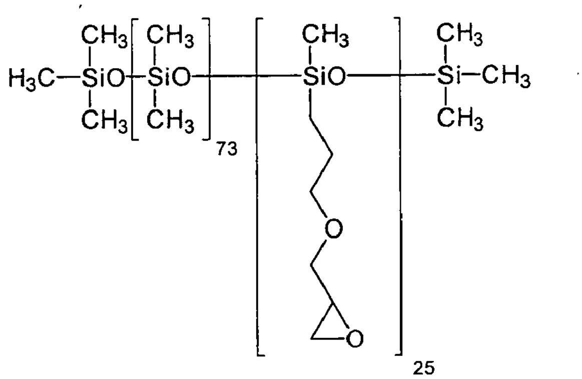

zugetropft, auf Rückflusstemperatur erwärmt und 6 Stunden gerührt. Anschließend wird bei 100 °C im Vakuum destilliert. Es wird ein hochviskoses, wachsartiges, gelb-braunes Produkt erhalten.

UV-vis Spektrum Beispiel 3 (0,0075 % in Isopropanol):

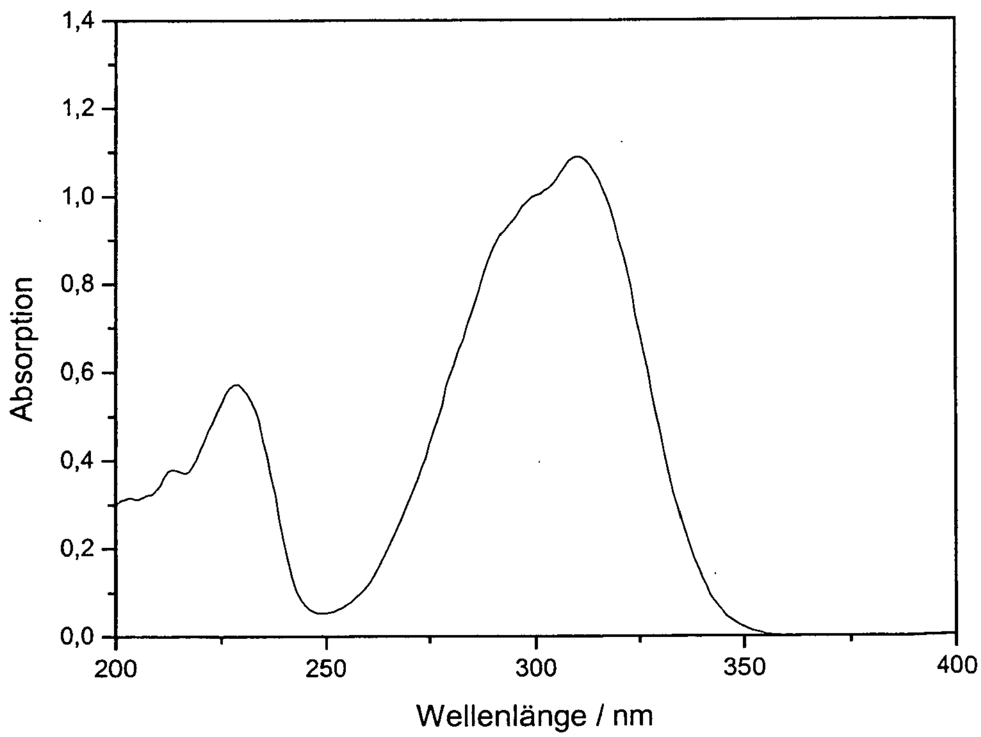


Das Spektrum zeigt die charakteristische Absorptionsbande der Methoxyzimtsäureeinheiten mit einem breiten Maximum zwischen 280 und 320 nm. Die Intensität korreliert mit der verwendeten Menge an Methoxyzimtsäure.

Anwendungstechnischer Vergleich:

Für den anwendungstechnischen Vergleich wurden folgende erfindungsgemäße UV-Licht absorbierende quaternäre Polysiloxane eingesetzt:

Verbindung 1:

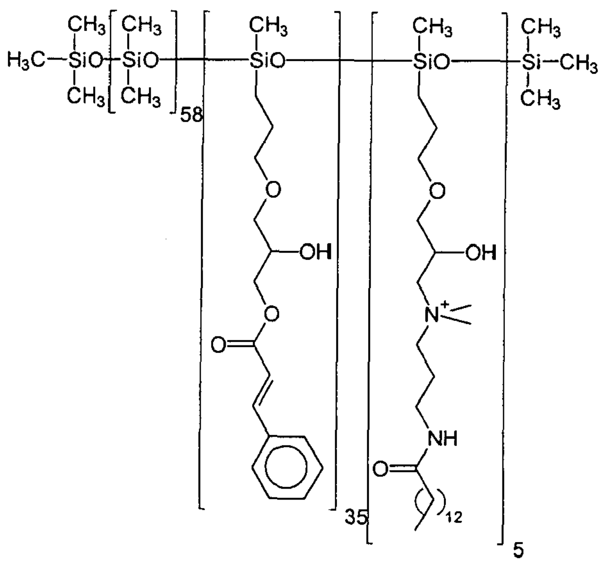

Verbindung 2:

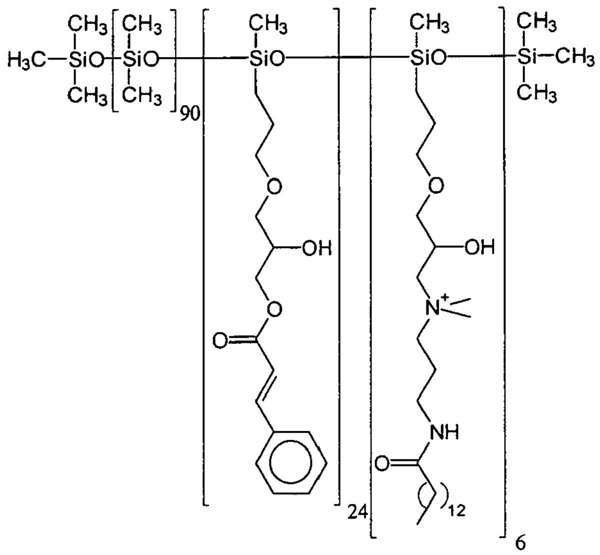

Verbindung 3:

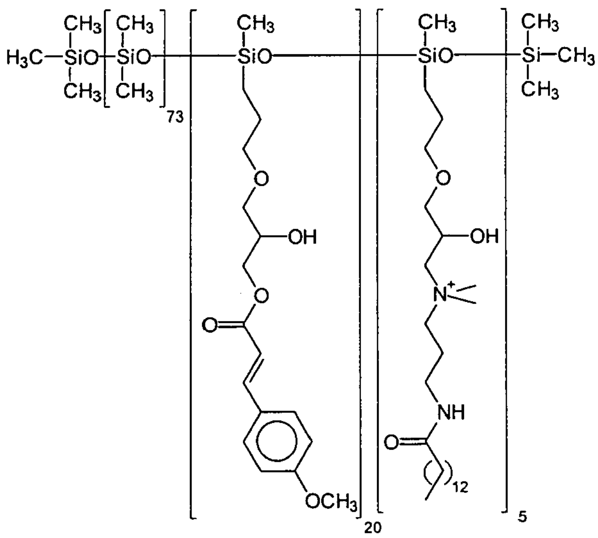

Herstellung und Überprüfung von Haarbehandlungsmitteln unter Verwendung der erfindungsgemäßen Verbindungen 1, 2 und 3:

Für die anwendungstechnische Beurteilung werden Haartressen, die für sensorische Tests verwendet werden, durch eine Dauerwellbehandlung und eine Bleichbehandlung standardisiert vorgeschädigt. Dazu werden friseurübliche Produkte verwendet.

<u>Materialien:</u>

- Dauerwell-Flüssigkeit (z.B. "ondi", Wella)
- Fixierung (z.B. "neutrafix", Wella)

- Blondierpulver (z.B. "blondor special", Wella)
- $H_2O_2$ (e.g. "Welloxyd 9 %", Wella)
- Shampoo ohne Pflegeanteil (z.B. Natriumlaurylethersulfat (12 % WAS), NaCl verdickt)
- Bechergläser
- Haar-Färbe-Pinsel

Die Behandlung wird in der folgenden Reihenfolge durchgeführt:

1. Dauerwell-Behandlung:

Die Haartressen werden mit der Dauerwellflüssigkeit angefeuchtet (Gewichtsverhältnis Haar : Flüssigkeit = 1 : 2).
Nach einer Einwirkzeit von 15 min bei Raumtemperatur in einem abgedeckten Becherglas wird die Dauerwellflüssigkeit 2 min sorgfältig ausgespült. Anschließend werden die Haarsträhnchen mit einem Handtuch sanft ausgedrückt.
Die Fixierung (Verhältnis Haar : Flüssigkeit = 1 : 2) hat eine Einwirkzeit von 10 min bei Raumtemperatur. Anschließend wird die Fixierung sorgfältig 2 min ausgespült.
Die Haare werden anschließend über Nacht bei Raumtemperatur getrocknet.

2. Bleich-Behandlung:

Das Blondierpulver und das $H_2O_2$ werden zu einer Paste (Gewichtsverhältnis Pulver: $H_2O_2$ = 2 : 3) verarbeitet. Dann wird die Paste sofort auf die dauergewellten Haare mit einem Pinsel aufgetragen. Die Einwirkzeit beträgt 30 min bei Zimmertemperatur. Anschließend wird die Blondierpaste unter fließendem Wasser 2 min ausgespült. Dann wird das Haar mit einem Shampoo ohne Conditioner 1 min gewaschen (Shampoomenge: 0,5 ml/Hairtress) und dann für 1 min ausgespült.
Bevor die vorgeschädigten Haarsträhnchen für Sensorik-Tests verwendet werden, werden sie über Nacht bei Raumtemperatur getrocknet.

Testrezeptur:

Die konditionierenden Produkte werden in einer einfachen Haarspülung mit folgendem Aufbau

| Produkt | Gewichtsanteile |
|---|---|
| TEGINACID® C Ceteareth-25 | 0,5 % |
| TEGO® Alkanol 16 Cetyl Alcohol | 2,0 % |
| "Konditioniermittel" | 2,0 % |
| Wasser | ad. 100 % |
| Zitronensäure | ad. pH 4,0 ± 0,3 |

getestet.

Als "Konditioniermittel" sind die erfindungsgemäßen Verbindungsbeispiele oder Vergleichsprodukte bezeichnet.

Standardisierte Behandlung von vorgeschädigten Haarsträhnen mit konditionierenden Proben:

Die, wie oben beschrieben, vorgeschädigten Haarsträhnchen werden wie folgt mit der oben beschriebenen konditionierenden Spülung behandelt:
Die Haarsträhnen werden unter fließendem, warmem Wasser benetzt. Das überschüssige Wasser wird leicht von Hand ausgedrückt, dann wird die Spülung aufgebracht und sanft im Haar eingearbeitet (1 ml/Haarsträhne (2 g)). Nach einer Verweilzeit von 1 min wird das Haar für 1 min gespült.

Vor der sensorischen Beurteilung wird das Haar an der Luft bei 50 % Luftfeuchtigkeit und 25 °C für mindestens 12 h getrocknet.

Beurteilungskriterien:

Die sensorischen Bewertungen erfolgen nach Noten, die auf einer Skala von 1 bis 5 vergeben werden, wobei 1 die schlechteste und 5 die beste Bewertung ist.

Nasskämmbarkeit:

| Bewertung | Zahnung des Kamms | Ergebnis |
|---|---|---|
| 5 | grob | keine Knoten, das Haar ist leicht entwirrbar |
| | fein | sehr leichtes Durchkämmen, kein Widerstand spürbar |
| 4 | grob | einzelne Knoten, das Haar ist leicht entwirrbar |
| | fein | leichtes Durchkämmen, geringer Widerstand spürbar |
| 3 | grob | wenige Knoten, geringer Widerstand |
| | fein | etwas Widerstand spürbar, der nach mehrmaligem Kämmen nachlässt |
| 2 | grob | einige Knoten, merklicher Widerstand |
| | fein | merklicher Widerstand, der nach mehrmaligem Kämmen nicht nachlässt. |
| 1 | grob | viele Knoten, starker Widerstand |
| | fein | sehr starker Widerstand, manchmal ist das Haar nicht durchkämmbar |

Nassgriff:

| Bewertung | Ergebnis |
|---|---|
| 5 | sehr glatt, weich aber trotzdem schön fest, griffig, nicht schmierig/klebrig (keine Rückstände fühlbar) |
| 4 | glatt und weich und/oder nur geringe Rückstände spürbar |
| 3 | glatt, etwas hart und/oder etwas Rückstände spürbar |
| 2 | hart und/oder merkliche schmierige, wachsige Rückstände |
| 1 | sehr hart, rauh, stumpf und/oder extrem schmierig, klebrig (deutlich spürbare schmierige, wachsige Rückstände spürbar) |

Trockenkämmbarkeit:

| Bewertung | Zahnung des Kamms | Ergebnis |
|---|---|---|
| 5 | grob | keine Knoten, das Haar ist leicht entwirrbar |
| | fein | sehr leichtes Durchkämmen, kein Widerstand spürbar, das Haar lädt sich nicht auf |
| 4 | grob | einzelne Knoten, das Haar ist leicht entwirrbar |
| | fein | leichtes Durchkämmen, geringer Widerstand spürbar, Haar lädt sich minimal auf |
| 3 | grob | wenige Knoten, geringer Widerstand |
| | fein | etwas Widerstand spürbar, der nach mehrmaligem Kämmen nachlässt, Haar lädt sich wenig auf |
| 2 | grob | einige Knoten, merklicher Widerstand |
| | fein | merklicher Widerstand, der nach mehrmaligem Kämmen nicht nachlässt, Haar lädt sich auf |
| 1 | grob | viele Knoten, starker Widerstand |

(fortgesetzt)

| Bewertung | Zahnung des Kamms | Ergebnis |
|---|---|---|
| | fein | sehr starker Widerstand, manchmal ist das Haar nicht durchkämmbar, Haar lädt sich deutlich auf |

Trockengriff:

| Bewertung | Ergebnis |
|---|---|
| 5 | sehr glatt, weich aber trotzdem fest, voll, griffig |
| 4 | glatt und weich |
| 3 | glatt, leicht hart und/oder leicht stumpf (Rückstände) |
| 2 | hart, etwas stumpf |
| 1 | rauh, hart, trocken, stumpf (Rückstände) |

Trocken-Aussehen:

| Bewertung | Ergebnis |
|---|---|
| 5 | extrem glänzend |
| 4 | glänzend |
| 3 | etwas glänzend |
| 2 | wenig glänzend, leicht stumpf |
| 1 | stumpf, kein Glanz |

In der folgenden Tabelle 1 werden die Ergebnisse der sensorischen Beurteilung der wie oben beschrieben durchgeführten Behandlung der Haarstränchen mit erfindungsgemäßen Substanzen bzw. Placebo verglichen.

Lichtbeständigkeit der Haarfarbe:

Zur Bestimmung der Lichtbeständigkeit der Haarfarbe werden gefärbte Haarsträhnen eingesetzt. Zur Färbung wurde ein handelsübliches Produkt der Marke Viva Farbton Grenadine Level 2 (Wella AG, Darmstadt) eingesetzt. Die Farbe der jeweiligen Haarsträhne wird zuerst vor der Behandlung mit der Haarspülung bestimmt. Nach der Behandlung wird die jeweilige Haarstähne mit einer Lampe bestrahlt deren Spektrum (D65) dem des natürlichen Sonnenlichtes weitestgehend entspricht. Die Dosis entsprach dabei der zweifachen natürlichen Tagesdosis bei 50 ° nördlicher Breite. Nach der Behandlung wird erneut eine Farbmessung vorgenommen. Die Auswertung der Farbmessung erfolgte nach dem CIE-L'a'b'-System, welches einen Differenzwert Delta-E ergibt. In dem Wert Delta-E werden Veränderungen der Helligkeit sowie auf der Rot-Grün-Skala und der Blau-Gelb-Skala berücksichtigt. Ein kleinerer Delta-E Wert steht für eine geringere Veränderung der Farbe.

Tabelle 1:

| Formulierung "einfache Haarspülung" mit | Nasskämmbarkeit | Nassgriff | Trockenkämmbarkeit | Trockengriff | Glanz | Farbveränderung Delta-E |
|---|---|---|---|---|---|---|
| Erfindungsgemäßer Verbindung 1 | 4,5 | 4,0 | 3,5 | 4,0 | 3,75 | 2,3 |
| Erfindungsgemäßer Verbindung 2 | 4,5 | 3,75 | 3,25 | 4,25 | 3,75 | 1,9 |
| Erfindungsgemäßer Verbindung 3 | 4,5 | 4,25 | 3,5 | 4,25 | 4,0 | 1,1 |
| Vergleichsverbindung Cetrimonium Chloride | 5,0 | 4,25 | 4,0 | 3,75 | 3,0 | 5,9 |
| Kontrolle ohne Konditioniermittel | 1,25 | 1,75 | 2,25 | 2,75 | 3,25 | n.b. |
| n.b. = nicht bestimmt | | | | | | |

Es zeigt sich, dass die erfindungsgemäßen Verbindungsbeispiele in der sensorischen Beurteilung sehr gute kosmetische Bewertungen erhalten.

Bei der Farbveränderung werden durch die erfindungsgemäßen Verbindungen signifikant niedrigere Werte als bei der Vergleichsverbindung beobachtet.

## Patentansprüche

**1.** UV-Licht absorbierende quaternäre Polysiloxane erhältlich durch Umsetzung von Verbindungen der allgemeinen Formel (II)

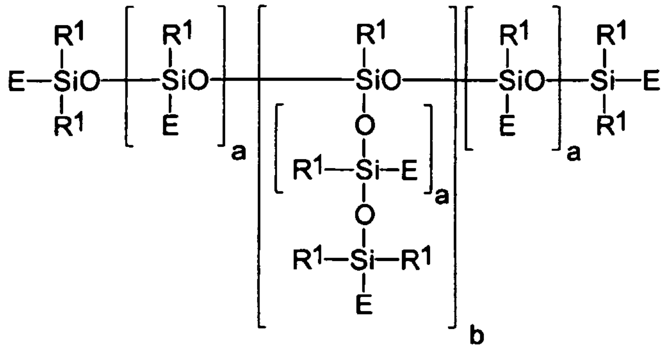

(II)

worin die Reste

$R^1$ gleich oder verschieden sind und jeweils Alkylreste mit 1 bis 22, insbesondere 1 bis 4 Kohlenstoffatomen oder Phenylreste bedeuten,
E zu einem Teil die Bedeutung der Reste $R^1$ haben können und die übrigen Reste E einwertige Reste sind, mit der allgemeinen Formel

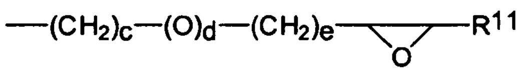

mit

c, e unabhängig voneinander 0 bis 10, insbesondere 1 bis 3, in der Summe ≥ 4,
d 0 oder 1 und
$R^{11}$ H oder ein einwertiger Kohlenwasserstoffrest mit 1 bis 10 Kohlenstoffatomen sein kann,

mit der Maßgabe, dass im durchschnittlichen Molekül mindestens zwei Reste E vorhanden sind,

a unabhängig voneinander einen Wert von 1 bis 200 und
b einen Wert von 0 bis 10 hat,

mit mindestens einer Verbindung der allgemeinen Formel (IIa)

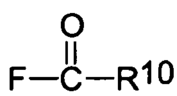

(IIa)

worin

F OH oder $NH_2$ sind,

$R^{10}$ ein einwertiger, für die UV-Absorption verantwortlicher Chromophorrest der Formel (Ic)

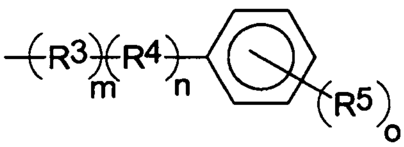

(Ic)

ist, worin

$R^3$

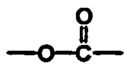

oder

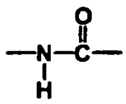

ist,

$R^4$ -CH=CH- ist,

$R^5$ gleich oder verschieden sind und jeweils Wasserstoff, Alkyl-, Haloalkyl-, Halogen-, Phenyl-, Hydroxyl-, Alkoxy-, Amino-, Alkylamino-, Dialkylamino-, Di(hydroxyalkyl)amino- oder Di(polyalkoxy)aminoreste bedeuten,

m = 0,

n = 0 oder 1,

o = 0 bis 5 ist,

und zusätzlich mit mindestens einem tertiären Amin der allgemeinen Formel (IIb)

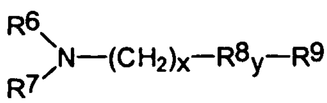

(IIb)

worin

$R^6$, $R^7$ Alkylreste mit 1 bis 22 Kohlenstoffatomen oder Alkenylreste mit 2 bis 22 Kohlenstoffatomen sind, wobei die Alkyl- oder Alkenylreste Hydroxylgruppen aufweisen können,

$R^8$

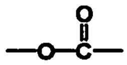

oder

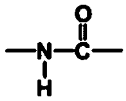

ist,
$R^9$ ein einwertiger Kohlenwasserstoffrest mit 1 bis 22 Kohlenstoffatomen oder ein Rest nach Formel (Ic),
x = 0 bis 6,
y = 0 oder 1 ist,

in an sich bekannter Weise in Gegenwart eines üblichen physiologisch verträglichen organischen oder anorganischen Säureäquivalentes HA, bezogen auf tertiäres Amin und bei Temperaturen von 40 bis 120 °C, wobei A- ein anorganisches oder organisches Anion, das von einer üblichen physiologisch verträglichen Säure HA herrührt, ist.

**2.** UV-Licht absorbierende Verbindungen gemäß Anspruch 1 **dadurch gekennzeichnet, dass** $R^1$ ein Methyl-, Ethyl-, n-Propyl-, iso-Propyl-, n-Butyl-, iso-Butyl- oder Phenylrest sein kann.

**3.** UV-Licht absorbierende Verbindungen gemäß den Ansprüchen 1 und 2 **dadurch gekennzeichnet, dass** A- gleich oder verschieden einer der Reste ist, ausgesucht aus der Gruppe Acetat-, Chlorid-, Bromid-, Hydrogensulfat-, Sulfat-, Methosulfat-, Ethosulfat-, Citrat-, Tartrat- und Lactationen, sowie Anionen aromatischer Säuren, wie die Anionen der p-Toluolsulfonsäure, Benzoesäure, Salicylsäure, Zimtsäure, 4-Methoxyzimtsäure, 4-Aminobenzoesäure, 4-Bis(hydroxypropyl)aminobenzoesäure, 4-Bis(polyethoxy)aminobenzoesäure, 4-Dimethylaminobenzoesäure, 3-Imidazol-4-yl-acrylsäure, 2-Phenylbenzimidazol-5-sulfonsäure, 3,3'-(1,4-Phenylendimethin)-bis(7,7-dimethyl-2-oxobicyclo[2.2.1]heptan-1-methansulfonsäure), 2-Hydroxy-4-methoxybenzophenon-5-sulfonsäure und 3-(4'-Sulfo)benzylidenbornan-2-on.

**4.** Verwendung der Verbindungen gemäß den Ansprüchen 1 bis 3 zur Herstellung von UV-Licht absorbierenden Formulierungen.

**5.** Verwendung der Verbindungen gemäß den Ansprüchen 1 bis 3 zur Herstellung von UV-Licht absorbierenden Formulierungen zur Behandlung, Nachbehandlung und Schutz von keratinischen Fasern sowie Haut und Hautanhangsgebilden.

**6.** Verwendung der Verbindungen gemäß den Ansprüchen 1 bis 3 zur Herstellung von UV-Licht absorbierenden Waschmittel- und Weichspülerformulierungen zur Behandlung und Nachbehandlung von Textilien.

## Claims

**1.** UV-light-absorbing quaternary polysiloxanes obtainable by reaction of compounds of the general formula (II)

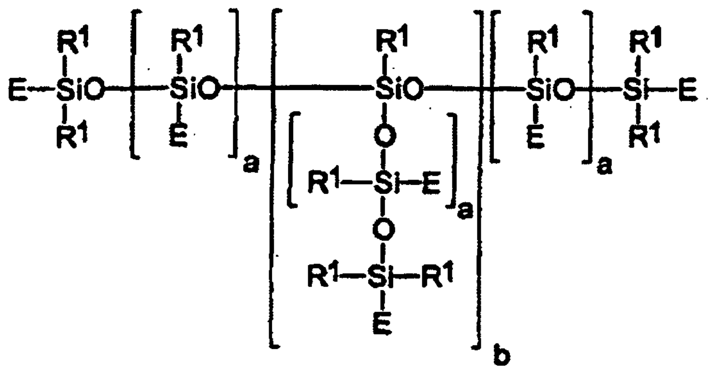

(II)

in which the radicals

$R^1$ are identical or different and are in each case alkyl radicals having 1 to 22, in particular 1 to 4, carbon atoms or phenyl radicals,
E can in part have the meaning of the radicals $R^1$ and the other radicals E are monovalent radicals with the general formula

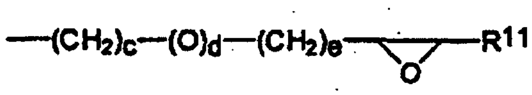

where

c, e independently of one another can be 0 to 10, in particular 1 to 3, in the sum of ≥ 4, d can be 0 or 1 and $R^{11}$ can be H or a monovalent hydrocarbon radical having 1 to 10 carbon atoms

with the proviso that, in the average molecule, at least two radicals E are present,

a independently of the others has a value of 1 to 200 and
b has a value of 0 to 10,

with at least one compound of the general formula (IIa)

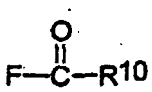

(IIa)

in which

F are OH or $NH_2$,
$R^{10}$ is a monovalent chromophore radical responsible for the UV absorption and of the formula (Ic),

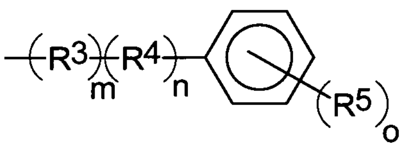

(Ic)

in which

$R^3$ is

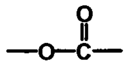

or

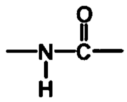

,

$R^4$ is -CH=CH-,

$R^5$ are identical or different and are in each case hydrogen, alkyl, haloalkyl, halogen, phenyl, hydroxyl, alkoxy, amino, alkylamino, dialkylamino, di(hydroxyalkyl)amino or di(polyalkoxy)amino radicals,

m = 0,

n = 0 or 1,

o = 0 to 5,

and additionally with at least one tertiary amine of the general formula (IIb)

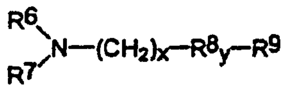

(IIb)

in which

$R^6$, $R^7$ are alkyl radicals having 1 to 22 carbon atoms or alkenyl radicals having 2 to 22 carbon atoms, in which the alkyl or alkenyl radicals can have hydroxyl groups,

$R^8$ is

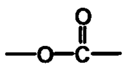

or

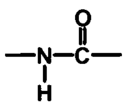

,

$R^9$ is a monovalent hydrocarbon radical having 1 to 22 carbon atoms or a radical according to formula (Ic),

x = 0 to 6,

y = 0 or 1

in a manner known per se in the presence of a conventional physiologically acceptable organic or inorganic acid equivalent HA, relative to tertiary amine and at temperatures from 40 to 120°C, A being an inorganic or organic anion resulting from a conventional physiologically acceptable acid HA.

**2.** UV-light-absorbing compounds according to Claim 1, **characterized in that** $R^1$ may be a methyl, ethyl, n-propyl, isopropyl, n-butyl, isobutyl or phenyl radical.

**3.** UV-light-absorbing compounds according to Claims 1 and 2, **characterized in that** $A^-$ is identical or different and is one of the radicals chosen from the group consisting of acetate, chloride, bromide, hydrogensulfate, sulfate, methosulfate, ethosulfate, citrate, tartrate and lactate ions, and anions of aromatic acids, such as the anions of p-toluenesulfonic acid, benzoic acid, salicylic acid, cinnamic acid, 4-methoxycinnamic acid, 4-aminobenzoic acid, 4-bis(hydroxypropyl)aminobenzoic acid, 4-bis(polyethoxy)aminobenzoic acid, 4-dimethylaminobenzoic acid, 3-imidazol-4-yl-acrylic acid, 2-phenylbenzimidazole-5-sulfonic acid, 3,3'-(1,4-phenylenedimethine)bis(7,7-dimethyl-2-oxo-bicyclo[2.2.1]heptane-1-methanesulfonic acid), 2-hydroxy-4-methoxybenzophenone-5-sulfonic acid and 3-(4'-sulfo) benzylidenebornan-2-one.

**4.** Use of the compounds according to Claims 1 to 3 for producing UV-light-absorbing formulations.

**5.** Use of the compounds according to Claims 1 to 3 for producing UV-light-absorbing formulations for the treatment, aftertreatment and protection of keratin fibres, and also skin and skin appendages.

**6.** Use of the compounds according to Claims 1 to 3 for producing UV-light-absorbing detergent and fabric softener formulations for the treatment and aftertreatment of textiles.

## Revendications

**1.** Polysiloxanes quaternaires absorbant la lumière UV pouvant être obtenus par la réaction de composés de formule générale (II)

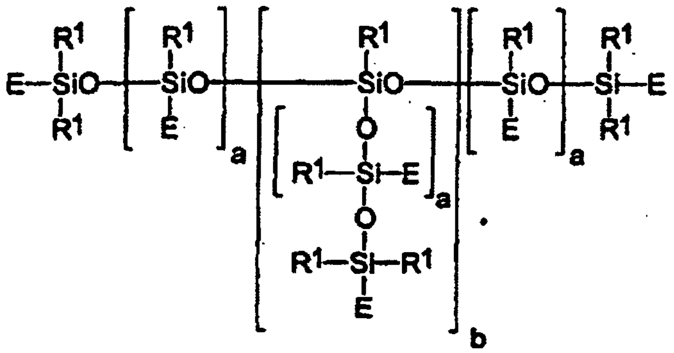

(II)

dans laquelle :

les radicaux $R^1$ sont identiques ou différents et signifient à chaque fois des radicaux alkyle comprenant 1 à 22, en particulier 1 à 4, atomes de carbone ou des radicaux phényle,
les radicaux E peuvent présenter pour une partie la signification des radicaux $R^1$ et les autres radicaux E sont des radicaux monovalents de formule générale

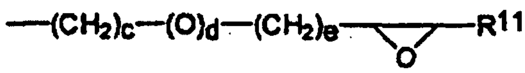

dans laquelle

c et e valent, indépendamment l'un de l'autre, de 0 à 10, en particulier de 1 à 3, d'une somme ≥ 4,
d vaut 0 ou 1, et
$R^{11}$ peut représenter un atome d'hydrogène ou un radical hydrocarboné monovalent comprenant 1 à 10 atomes de carbone,

à condition que, dans la molécule moyenne, au moins deux radicaux E sont présents,

a vaut indépendamment de 1 à 200, et
b vaut de 0 à 10,

avec au moins un composé de formule générale (IIa)

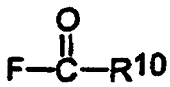 (IIa)

dans laquelle :

F représente un groupe OH ou un groupe $NH_2$,
$R^{10}$ représente un radical chromophore monovalent responsable de l'absorption UV, de formule (Ic)

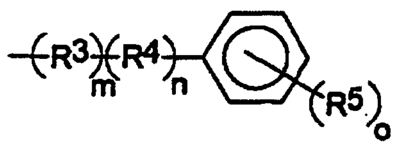 (Ic)

dans laquelle:

$R^3$ représente

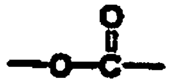

ou

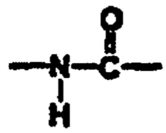

,

$R^4$ représente -CH=CH-,
les $R^5$ sont identiques ou différents et représentent, chacun, un hydrogène, des radicaux alkyle, halogénoalkyle, halogéno, phényle, hydroxyle, alcoxy, amino, alkylamino, dialkylamino, di(hydroxyalkyl)amino ou di(polyalcoxy)amino,
m vaut 0,
n vaut 0 ou 1,
o vaut de 0 à 5,

et, en outre, avec au moins une amine tertiaire de formule générale (IIb)

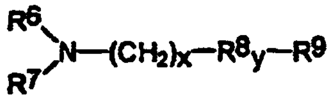

(IIb)

dans laquelle :

$R^6$ et $R^7$ représentent des radicaux alkyle comprenant de 1 à 22 atomes de carbone ou des radicaux alcényle comprenant de 2 à 22 atomes de carbone, les radicaux alkyle ou alcényle pouvant renfermer des groupes hydroxyle,
$R^8$ représente

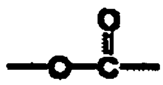

ou

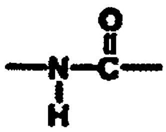

,

$R^9$ représente un radical hydrocarboné monovalent comprenant de 1 à 22 atomes de carbone ou un radical selon la formule (Ic),
x vaut de 0 à 6
y vaut 0 ou 1,

d'une façon connue en soi, en présence d'un équivalent d'acide HA habituel, organique ou inorganique, physiologiquement acceptable, par rapport à l'amine tertiaire, et à des températures de 40 à 120 °C, où A- est un anion inorganique ou organique dérivé d'un acide HA habituel physiologiquement acceptable.

**2.** Composés absorbant la lumière UV selon la revendication 1, **caractérisés en ce que** $R^1$ peut être un radical

méthyle, éthyle, n-propyle, iso-propyle, n-butyle, iso-butyle ou phényle.

3. Composés absorbant la lumière UV selon les revendications 1 et 2, **caractérisés en ce que** A- est identique ou différent et représente un des radicaux, choisi dans le groupe constitué par les ions acétate, chlorure, bromure, hydrogénosulfate, sulfate, méthosulfate, éthosulfate, citrate, tartrate et lactate, ainsi que les anions d'acides aromatiques, tels que les anions de l'acide p-toluènesulfonique, l'acide benzoïque, l'acide salicylique, l'acide cinnamique, l'acide 4-méthoxycinnamique, l'acide 4-aminobenzoïque, l'acide 4-bis(hydroxypropyl)aminobenzoïque, l'acide 4-bis-(polyéthoxy)aminobenzoïque, l'acide 4-diméthylaminobenzoïque, l'acide 3-imidazol-4-yl-acrylique, l'acide 2-phénylbenzimidazole-5-sulfonique, l'acide 3,3'-(1,4-phénylènediméthine)-bis(7,7-diméthyl-2-oxo-bicyclo[2.2.1] heptane-1-méthanesulfonique), l'acide 2-hydroxy-4-méthoxybenzophénone-5-sulfonique et la 3-(4'-sulfo)benzylidène-bornan-2-one.

4. Utilisation des composés selon les revendications 1 à 3 pour la production de formulations absorbant la lumière UV.

5. Utilisation des composés selon les revendications 1 à 3 pour la production de formulations absorbant la lumière UV pour le traitement, le post-traitement et la protection de fibres kératiniques, ainsi que de la peau et d'annexes de la peau.

6. Utilisation des composés selon les revendications 1 à 3 pour le production de formulations détergentes et adoucissantes absorbant la lumière UV pour le traitement et le post-traitement de textiles.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0282720 A
- DE 3719086 A
- WO 0125385 A
- WO 0125382 A
- WO 0125381 A
- WO 0125380 A
- WO 9955953 A
- EP 0282720 B
- DE 3719086 C
- DE 10141356 A
- US 6630132 B